# EUROPEAN PATENT SPECIFICATION

(11) **EP 4 088 653 B1**
(45) Date of publication and mention of the grant of the patent: **16.09.2026**
(21) Application number: 22165867.7
(22) Date of filing: 31.03.2022
(51) Int. Cl.: A61B 5/022, A61B 5/026, G16H 50/20, G16H 50/30, G16H 40/63, A61B 5/024, A61B 5/08, A61B 5/1455

(54) **PULSE WAVE ANALYSIS DEVICE, PULSE WAVE ANALYSIS METHOD, AND PULSE WAVE ANALYSIS PROGRAM**
PULSWELLENANALYSEVORRICHTUNG, PULSWELLENANALYSEVERFAHREN UND PULSWELLENANALYSEPROGRAMM
DISPOSITIF D'ANALYSE D'ONDE PULSÉE, PROCÉDÉ D'ANALYSE D'ONDE PULSÉE ET PROGRAMME D'ANALYSE D'ONDE PULSÉE

(30) Priority: 23.04.2021 JP 2021073049
(43) Date of publication of application: 16.11.2022
(73) Proprietor: Nihon Kohden Corporation, Shinjuku-ku Tokyo (JP)
(72) Inventor: Morimoto, Haruka, Saitama, 359-0037 (JP); Kobayashi, Naoki, Saitama, 359-0037 (JP); Motogi, Jun, Saitama, 359-0037 (JP)
(74) Representative: Grünecker Patent- und Rechtsanwälte PartG mbB

(56) References cited:
- EP-A1- 2 912 996
- WO-A1-2020/264439
- US-A1- 2015 119 653
- US-A1- 2017 273 582

## Description

### TECHNICAL FIELD

The presently disclosed subject matter relates to a pulse wave analysis device, a pulse wave analysis method, and a pulse wave analysis program.

### BACKGROUND ART

In recent years, patients with cardiac diseases including heart failure have increased. In particular, it is expected that the number of elderly patients with the heart failure will increase significantly in the future as the number of elderly people increases. In addition, there are many cases in which the patient is hospitalized for treatment of heart failure, is discharged after completing necessary treatment, and then is re-hospitalized within a short period of time, and an increase in medical expenses and the like becomes a problem at home and abroad.

A cause of rehospitalization for the heart failure patient is said to be mainly congestion. An example of the congestion is congestion in body veins (body congestion) due to an increase in a right atrial pressure. Since blood is not sufficiently circulated due to a decrease in a cardiac function, the blood is stagnant in an organ or tissue of a body, and the congestion is generated. When the congestion occurs in a body venous system, a central venous pressure increases, and thus an extent of the congestion can be grasped by measuring the central venous pressure. From a viewpoint of measurement accuracy, an invasive measurement method of the central venous pressure using a catheter has been adopted in the past, but since the measurement is invasive, it imposes a heavy burden on the patient and cannot be easily performed. In addition, as a simple estimation method instead of the invasive measurement method, there is a method in which a doctor sees distension of a jugular vein, but a procedure is largely based on an experience of the doctor and cannot be measured quantitatively.

Therefore, if the central venous pressure can be measured noninvasively and quantitatively and accurately, it is possible to easily grasp a congestive state of the heart failure patient in a ward, a clinic, or even at home. For example, Patent Literature 1 below discloses a technique for calculating a central venous pressure from a pulse wave of a patient measured noninvasively based on a principle of an oscillometric method. However, measurement of the central venous pressure is likely to be affected by respiration, and with the technique described above, there is room for further improvement in measurement accuracy in a case where the patient is in a congestive state and in a state where it is difficult to breathe. In relation to this, Patent Literatures 2 and 3 below disclose a technique for calculating an index indicating respiratory variation in an arterial pressure from a pulse wave of a patient measured noninvasively.

On the other hand, the heart failure patient often has arrhythmia in many cases, and there is room for further improvement in the measurement of the central venous pressure in the measurement using the oscillometric method for a patient with arrhythmia.

In addition, the heart failure patient often has abnormal respiration and pulse wave due to unstable hemodynamics. A respiratory state of the patient is often determined by findings of the doctor, and the doctor grasps the respiratory state from respiration and pulse of the patient, and makes a diagnosis based on a diagnostic criterion.

Thus, in a present situation, there is room for further improvement in accuracy in a noninvasive measurement of the central venous pressure depending on a state of the patient, and a medical worker needs to grasp a measurement result and accuracy (or reliability) of the measurement result.

### CITATION LIST

### PATENT LITERATURE

Patent Literature 1: Japanese Patent No. 5694032
Patent Literature 2: Japanese Patent No. 6522327
Patent Literature 3: Japanese Patent No. 6282887
EP 2 912 996 A1 discloses a blood pressure measuring apparatus and a blood pressure measuring method.
WO 2020/264439 A1 teaches a medical apparatus, systems and methods for non-invasive measurement of cardiovascular parameters.

### SUMMARY OF THE INVENTION

According to the invention there are provided a pulse wave analysis device, method and program according to claims 1, 13 and 14, preferred embodiments being specified in dependent claims 2-12

### SUMMARY OF THE DISCLOSURE

The presently disclosed subject matter has been made to solve the above-described problems. Therefore, a main object of the presently disclosed subject matter is to provide a pulse wave analysis device, a pulse wave analysis method, and a pulse wave analysis program capable of measuring a central venous pressure of a patient noninvasively and notifying a medical worker of accuracy of a measurement result.

The above object of the presently disclosed subject matter is achieved by the following.
(1) A pulse wave analysis device includes a pulse wave acquisition unit, a venous pressure calculation unit, a parameter calculation unit, and an output. The pulse wave acquisition unit acquires a pulse wave of a living body. The venous pressure calculation unit calculates a venous pressure based on a waveform of the pulse wave in a predetermined period. The parameter calculation unit analyzes the waveform in the predetermined period and calculates at least one vital parameter for the living body. The output outputs a calculated venous pressure, a calculated vital parameter, and/or information on accuracy of the venous pressure based on the vital parameter.
(2) A pulse wave analysis method includes: acquiring a pulse wave of a living body; calculating a venous pressure based on a waveform of the pulse wave in a predetermined period; analyzing the waveform in the predetermined period and calculating at least one vital parameter for the living body; and outputting a calculated venous pressure, a calculated vital parameter, and/or information on accuracy of the venous pressure based on the vital parameter.
(3) A pulse wave analysis program that causes a computer to execute a processing including: acquiring a pulse wave of a living body; calculating a venous pressure based on a waveform of the pulse wave in a predetermined period; analyzing the waveform in the predetermined period and calculating at least one vital parameter for the living body; and outputting a calculated venous pressure, a calculated vital parameter, and/or information on accuracy of the venous pressure based on the vital parameter.

According to the presently disclosed subject matter, since a measured central venous pressure, the vital parameter, and/or information on accuracy of the central venous pressure based on the vital parameter are output, the central venous pressure of a patient and the accuracy of the measured central venous pressure can be notified to a medical worker. Therefore, the medical worker can confirm the measured central venous pressure with recognizing measurement accuracy.

### BRIEF DESCRIPTION OF DRAWINGS

FIG. 1 is a block diagram illustrating a schematic hardware configuration of a pulse wave analysis system according to an embodiment.
FIG. 2 is a block diagram illustrating a schematic hardware configuration of a controller illustrated in FIG. 1.
FIG. 3 is a functional block diagram illustrating main functions of the controller illustrated in FIG. 1.
FIG. 4 is a flow chart for explaining a processing procedure of a pulse wave analysis method of a pulse wave analysis device illustrated in FIG. 1.
FIG. 5A is a diagram illustrating a variation over time in a cuff pressure applied to an upper arm of a patient, and FIG. 5B is a diagram illustrating pulse wave data extracted from the variation over time in the cuff pressure in FIG. 5A.
FIG. 6 is a waveform diagram illustrating a waveform of a pulse wave for each step of the pulse wave data of FIG. 5B.
FIG. 7 is a diagram illustrating a result of frequency analysis of the waveform of the pulse wave for each step illustrated in FIG. 6.
FIG. 8 is a waveform diagram illustrating the waveform of the pulse wave for each step of the pulse wave including a respiratory component.
FIG. 9 is a diagram illustrating a result of the frequency analysis of the pulse wave for each step illustrated in FIG. 8.
FIG. 10 is a waveform diagram illustrating the waveform of the pulse wave including arrhythmia for each step.
FIG. 11 is a diagram illustrating a result of the frequency analysis of the pulse wave for each step illustrated in FIG. 10.
FIG. 12 is a schematic diagram illustrating a case where a measurement result of a central venous pressure and information on accuracy of the measurement result are displayed on a monitor screen.
FIG. 13A is a diagram illustrating the variation over time in the cuff pressure applied to the upper arm of the patient, and FIG. 13B is a diagram illustrating the pulse wave data extracted from the variation over time in the cuff pressure in FIG. 13A.
FIG. 14 is a waveform diagram illustrating the waveform of the pulse wave for each step of the pulse wave data illustrated in FIG. 13B.
FIG. 15 is a diagram illustrating a result of the frequency analysis of the pulse wave for each step illustrated in FIG. 14.
FIG. 16 is a schematic diagram illustrating a case where the measurement result of the central venous pressure and a respiration depth as a vital parameter are displayed on the monitor screen.
FIG. 17 is a graph illustrating power of respiration for each step.
FIG. 18 is a graph illustrating a heart beat component/the respiratory component for each step.
FIG. 19A is a diagram illustrating the variation over time in the cuff pressure applied to the upper arm of the patient, and FIG. 19B is a diagram illustrating the pulse wave data extracted from the variation over time in the cuff pressure in FIG. 19A.
FIG. 20 is a waveform diagram illustrating the waveform of the pulse wave for each step of the pulse wave data illustrated in FIG. 19B.
FIG. 21 is a diagram illustrating a result of the frequency analysis of the pulse wave for each step illustrated in FIG. 20.
FIG. 22 is a schematic diagram illustrating a case where the measurement result of the central venous pressure and presence or absence of arrhythmia as the vital parameter are displayed on the monitor screen.

### DESCRIPTION OF EMBODIMENTS

Hereinafter, an embodiment of the presently disclosed subject matter will be described with reference to the accompanying drawings. In the drawings, the same members are denoted by the same reference signs. In addition, dimensional ratios in the drawings are exaggerated for convenience of explanation and may differ from actual ratios.

### <PULSE WAVE ANALYSIS SYSTEM 100>

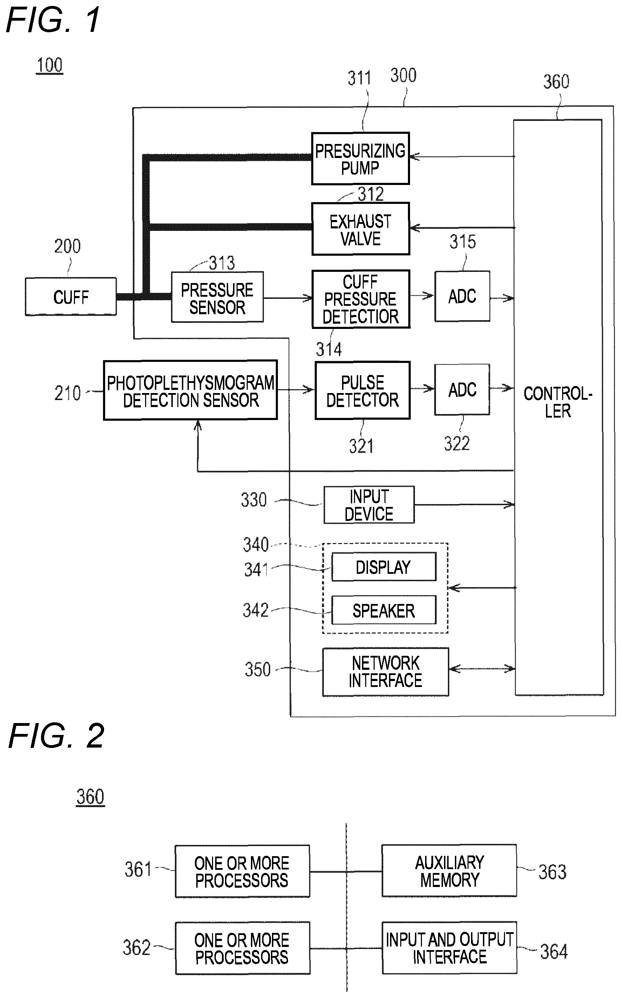

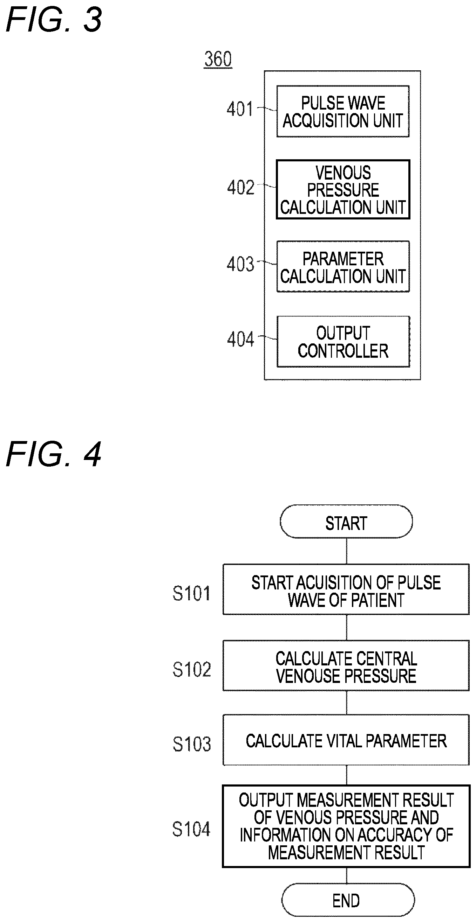

FIG. 1 is a block diagram illustrating a schematic hardware configuration of the pulse wave analysis system 100 according to an embodiment. FIG. 2 is a block diagram illustrating a schematic hardware configuration of a controller 360 illustrated in FIG. 1. FIG. 3 is a functional block diagram illustrating main functions of the controller 360 illustrated in FIG. 1.

The pulse wave analysis system 100 of the present embodiment is a system that noninvasively measures a central venous pressure of a patient, and outputs accuracy of a measured central venous pressure (hereinafter, also simply referred to as "venous pressure") in addition to a measurement result of the central venous pressure.

The central venous pressure is an important index for a doctor to grasp hemodynamics of the patient. The central venous pressure is an index reflecting a pressure measured by a right atrium (right atrial pressure), and can reflect an amount of blood returning to a heart and a preload of the right atrium. Therefore, since the central venous pressure reflects an amount of circulating blood, the doctor can evaluate body congestion from an increase of the central venous pressure. Congestion is a main cause of exacerbation of heart failure, in which case the central venous pressure is known to be increased.

However, in the past, the central venous pressure is generally measured invasively using a Swan-Ganz catheter, for example. In contrast, in the present embodiment, by acquiring a pulse wave of the patient and estimating the central venous pressure from the pulse wave, it is possible to avoid invasively measuring the central venous pressure of the patient, and it is possible to noninvasively measure the central venous pressure. Accordingly, a burden on the patient with respect to measurement of the central venous pressure can be significantly reduced.

As illustrated in FIG. 1, the pulse wave analysis system 100 can include a cuff 200, a photoplethysmogram detection sensor 210, and a pulse wave analysis device 300. The cuff 200 is configured to be connectable to the pulse wave analysis device 300, and is worn by winding an air bag around an upper arm of the patient. The photoplethysmogram detection sensor 210 will be described later.

### <Pulse Wave Analysis Device 300>

The pulse wave analysis device 300 may be a medical device (for example, a patient monitor or the like) that analyzes physiological information of the patient and displays an analysis result. A main configuration of the pulse wave analysis device 300 will be described below.

The pulse wave analysis device 300 can include a pressurizing pump 311, an exhaust valve 312, a pressure sensor 313, a cuff pressure detector 314, an AD converter (ADC) 315, a pulse detector 321, an ADC 322, an input device 330, an output device 340, a network interface 350, and the controller 360.

The pressurizing pump 311 feeds air to the air bag of the cuff 200 in response to an instruction from the controller 360, and increases a pressure in the air bag (hereinafter referred to as "cuff internal pressure"). This can increase a compression pressure (hereinafter referred to as "cuff pressure") applied to the upper arm in the vicinity of an axilla of the patient by the cuff 200. In addition, the exhaust valve 312 releases air in the air bag to the atmosphere, thereby gradually exhausting the air from the cuff 200 and reducing the cuff internal pressure. Accordingly, the cuff pressure can be reduced.

The pressure sensor 313 detects the cuff pressure. A pulse wave (cuff pulse wave) of the patient in a process of increasing and reducing the cuff pressure is superimposed on the cuff pressure. The cuff pressure detector 314 extracts the pulse wave superimposed on the cuff pressure from a detected cuff pressure, and outputs the cuff pressure and the extracted pulse wave to the ADC 315 as an analog signal. The ADC 315 converts the analog signals of the cuff pressure and the pulse wave into digital signals and transmits the digital signals to the controller 360. The pressure sensor 313, the cuff pressure detector 314, and the ADC 315 may be integrated with the cuff 200. In this case, the controller 360 receives the digital signal of the pulse wave. Same or similarly, the pressure sensor 313 and the cuff pressure detector 314 may be integrated with the cuff 200, and the ADC 315 may be included in the pulse wave analysis device 300.

In the present embodiment, the cuff 200, the pressure sensor 313, the cuff pressure detector 314, and the ADC 315 function as a pulse wave measurer and measure the pulse wave of the patient.

The photoplethysmogram detection sensor 210 (hereinafter, referred to as a "pulse wave sensor 210"), the pulse detector 321, and the ADC 322 function as a photoplethysmogram measurer, detect the pulse wave of the patient using a photoplethysmogram method, and transmit data of the detected pulse wave to the controller 360. The pulse detector 321 and the ADC 322 may be built in the pulse wave sensor 210, and the pulse wave sensor 210 may transmit pulse wave data to the controller 360. Same or similarly, only the ADC 322 may be included in the pulse wave analysis device 300.

The pulse wave sensor 210 is an SpO2 probe for measuring arterial oxygen saturation (SpO2), and is put on extremities (for example, a tip of a finger) of a body of the patient. In the present embodiment, it is preferable that the pulse wave sensor 210 is put on the tip of the finger of the patient, but the presently disclosed subject matter is not limited thereto.

The pulse wave sensor 210 can include a light emitter and a light detector, and transmits red light or infrared light to the patient at a predetermined light emission timing, and detects transmitted light. The light emitter can include, for example, a light emitting diode that emits light with a wavelength in the vicinity of 660 nm (red) or in the vicinity of 940 nm (infrared), and emits light toward a living body surface (tip of the finger) of the patient. The light detector can include, for example, a photodiode, detects the transmitted light transmitted through a blood vessel and a biological tissue, and converts the transmitted light into an electrical signal corresponding to the transmitted light.

The pulse detector 321 detects the pulse wave from the electrical signal generated by the pulse wave sensor 210, and outputs the pulse wave as a photoplethysmogram signal to the ADC 322. The ADC 322 converts the photoplethysmogram signal into a digital signal and outputs the digital signal to the controller 360.

The input device 330 is configured to receive an input operation of a user such as a medical worker who operates the pulse wave analysis device 300, and to generate an input signal corresponding to the input operation. The input device 330 can include, for example, a touch panel superimposed on a display 341 of the output device 340, an operation button attached to a housing of the pulse wave analysis device 300, a mouse, a keyboard, and the like. The input signal generated by the input device 330 is transmitted to the controller 360, and the controller 360 executes a predetermined processing according to the input signal.

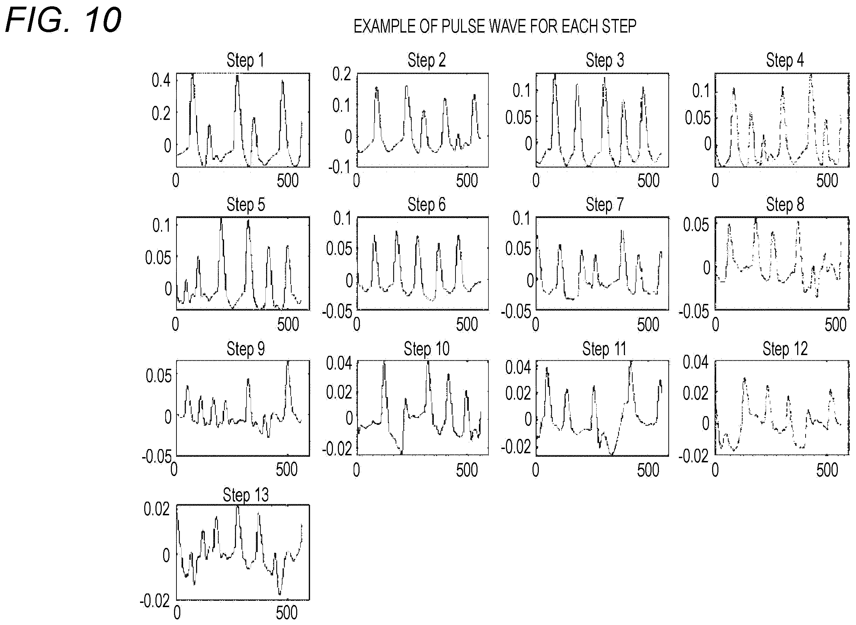

The output device 340 functions as an output, and in the present embodiment, outputs the measurement result of the central venous pressure and information on accuracy of the measurement result. The output device 340 can include the display 341 and a speaker 342. The display 341 functions as a display, and may be a liquid crystal display, an organic EL display, or the like attached to the housing of the pulse wave analysis device 300. The display 341 may be a display device such as a transmissive or non-transmissive head mounted display worn on a head of the user. The display 341 displays, for example, the measurement result of the venous pressure and the information on the accuracy of the measurement result (see FIG. 10).

The speaker 342 is attached to the housing of the pulse wave analysis device 300, and can output the measurement result of the venous pressure and the information on the accuracy of the measurement result with a sound. In addition, the speaker 342 can issue an alarm to the user by sound. In addition, the output device 340 may include a light emitter including an LED or the like, and may be configured to emit an alert by light such as an LED.

The output device 340 is not limited to the display 341, the speaker 342, and the LED, and may include, for example, a printer for printing and outputting the measurement result of the venous pressure and the accuracy of the measurement result.

The network interface 350 is configured to connect the controller 360 to a communication network. Specifically, the network interface 350 can include a processing circuit for various interfaces for communicating with an external device such as a server via a communication network, and is configured to conform to a communication standard for communicating via the communication network. The communication network is a local area network (LAN), a wide area network (WAN), the Internet, or the like.

The controller 360 measures the venous pressure of the patient, and outputs the measurement result of the venous pressure and the information on the accuracy of the measurement result. The controller 360 may be software and hardware for governing main control of the pulse wave analysis device 300, or the controller 360 may be an independent device. For example, the controller 360 may be a dedicated medical device that performs pulse wave analysis of the patient, or may be a personal computer, a smartphone, a tablet terminal, or the like in which a pulse wave analysis program for performing the pulse wave analysis is installed. Further, the controller 360 may be a wearable device or the like worn on the body (for example, an arm, a head, or the like) of the user.

As illustrated in FIG. 2, the controller 360 can include one or more processors (central processing unit: CPU) 361, one or more memories 362, an auxiliary memory 363, and an input and output interface 364.

The one or more memories 362 can include a read only memory (ROM) and a random access memory (RAM). The ROM stores various programs, parameters, and the like. The RAM can include a work area in which various programs and the like executed by the one or more processors 361 are stored. The one or more processors 361 are configured to expand a program specified by various programs stored in the ROM or the auxiliary memory 363 onto the RAM, and execute various processing in cooperation with the RAM. The RAM stores the measurement result of the venous pressure, the information on the accuracy of the measurement result, an appropriate range of the venous pressure, a calculation result of a vital parameter, a cut-off value of the vital parameter, an appropriate range, and the like. The RAM functions as a first memory and a second memory.

The auxiliary memory 363 can include, for example, a storage device (storage) such as a hard disk drive (HDD), a solid state drive (SSD), or a USB flash memory. The auxiliary memory 363 is configured to store the pulse wave analysis program and various types of data. The auxiliary memory 363 stores the pulse wave data acquired by a pulse wave acquisition unit 401. Here, the program can be stored using various types of non-transitory computer-readable media and supplied to a computer. The non-transitory computer-readable medium includes various types of tangible storage media.

The input and output interface 364 functions as an interface between the one or more processors 361 and the input device 330 and an output device 350. The input and output interface 364 can include various communication modules that communicate with an input device such as a mouse and a keyboard, a drive module that drives the display 341 and the speaker 342, and the like.

When the one or more processors 361 execute the pulse wave analysis program, the controller 360 controls each unit of the pulse wave analysis device 300 to implement various functions.

FIG. 3 is a functional block diagram illustrating main functions of the controller 360. The controller 360 can include the pulse wave acquisition unit 401, a venous pressure calculation unit 402, a parameter calculation unit 403, and an output controller 404. Hereinafter, each function of the controller 360 will be described in detail with reference to FIG. 4.

### <Pulse Wave Analysis Method>

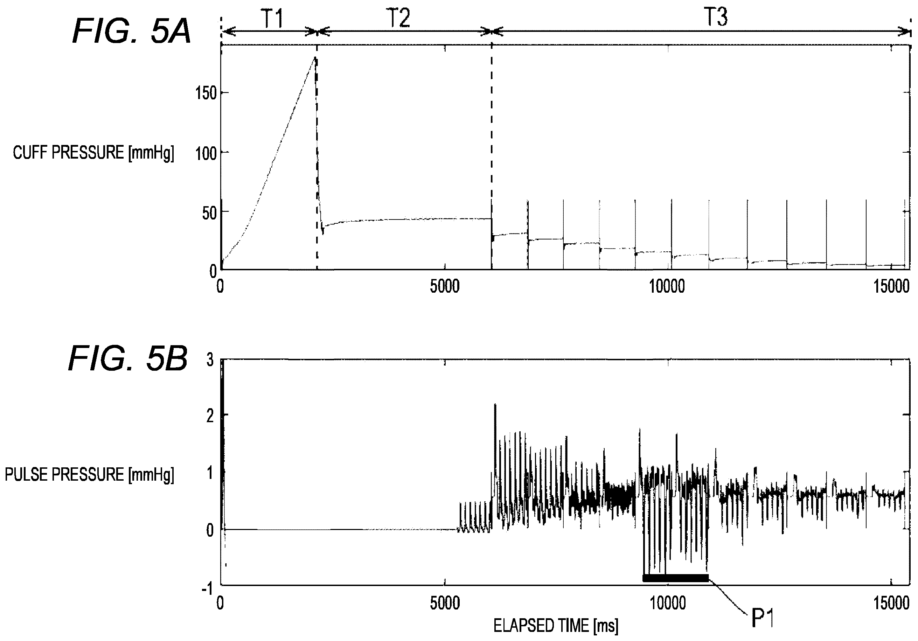

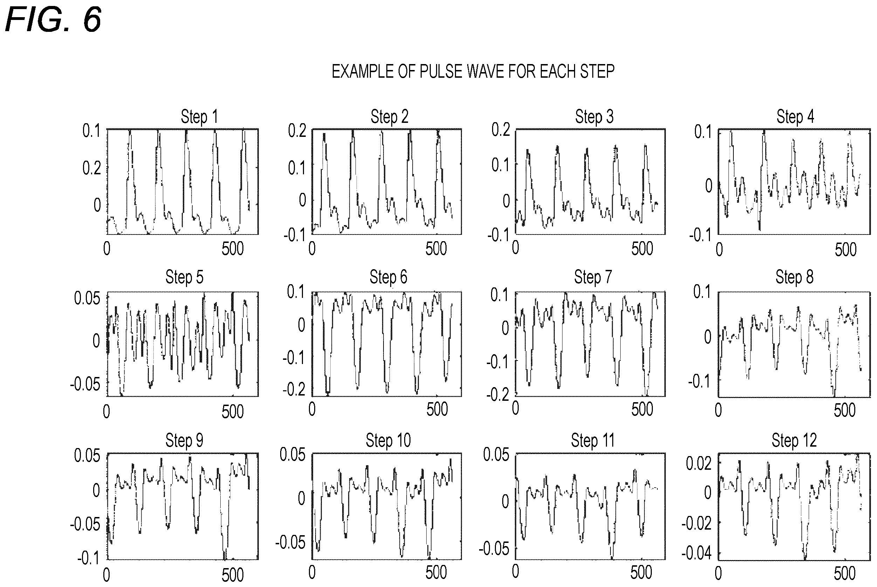

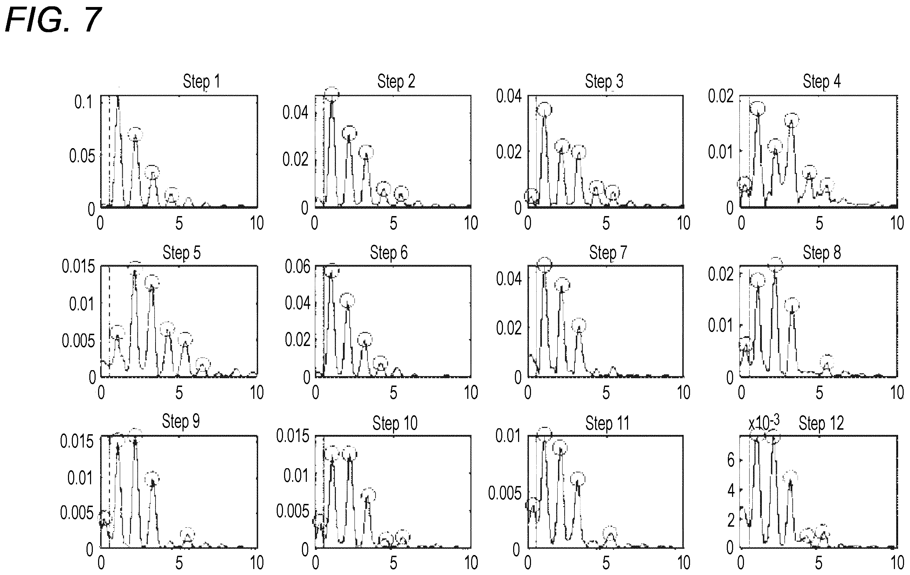

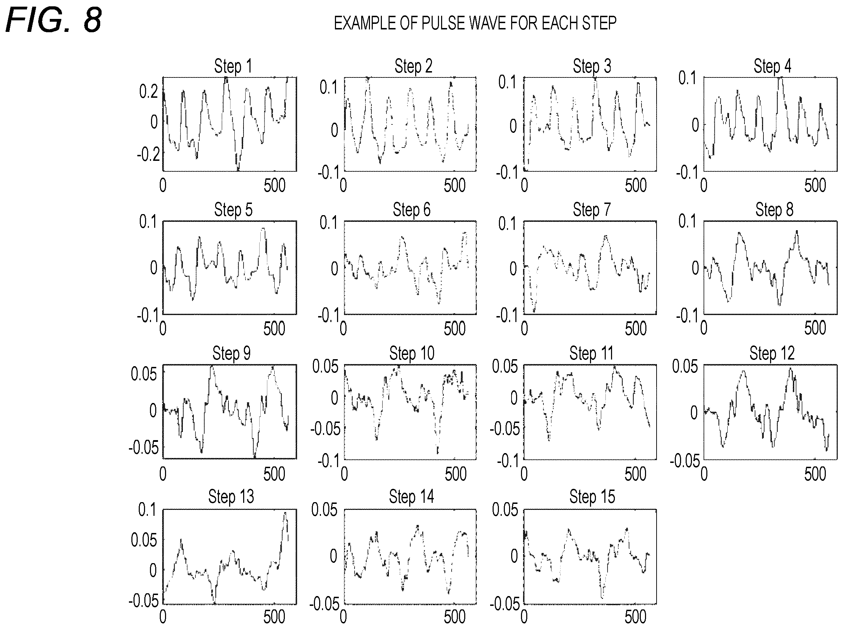

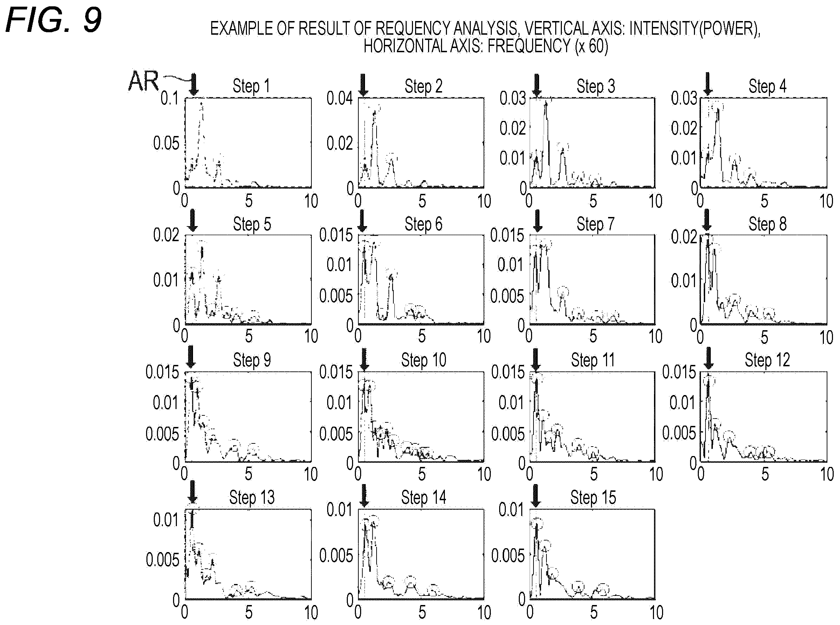

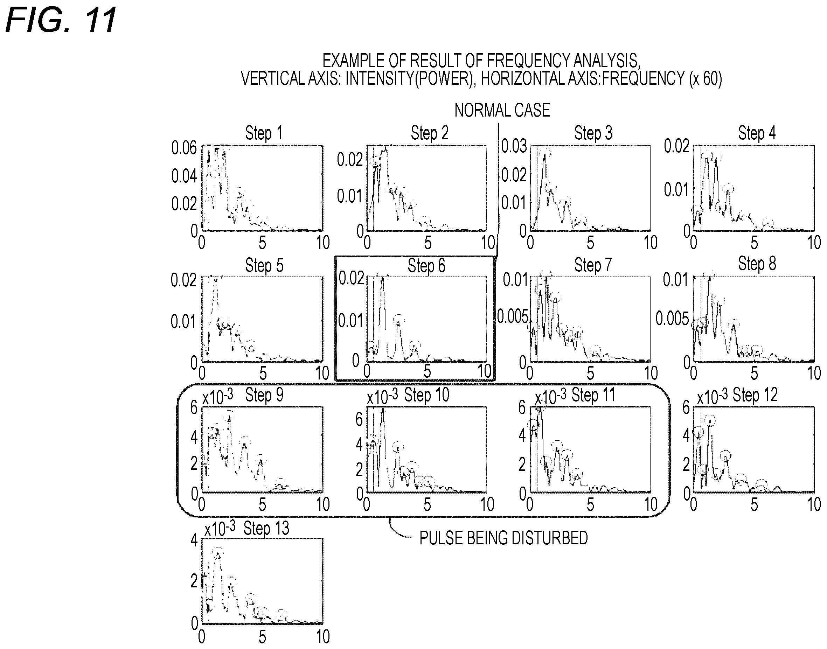

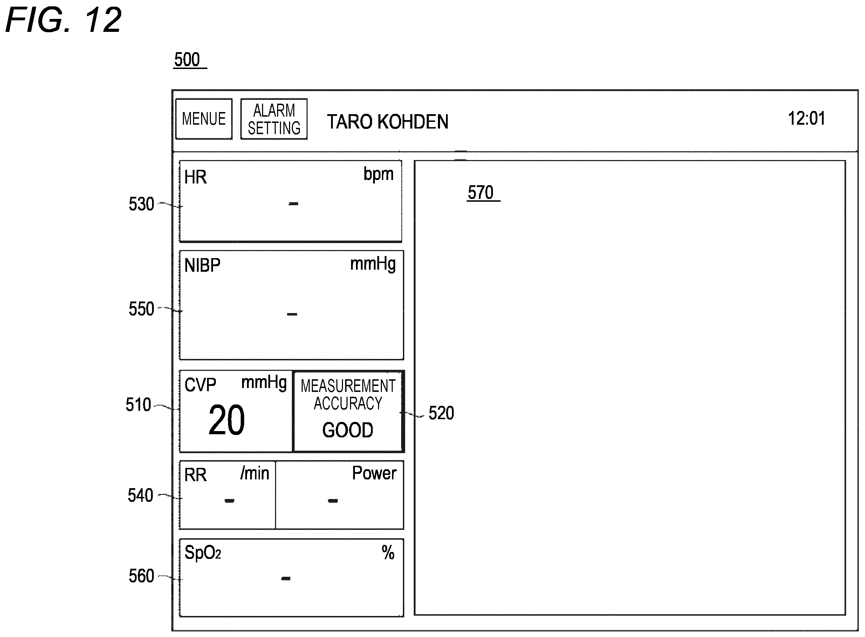

FIG. 4 is a flow chart for explaining a processing procedure of the pulse wave analysis method of the pulse wave analysis device 300. The processing of the flow chart in the figure is implemented by the one or more processors 361 executing the pulse wave analysis program. FIG. 5A is a diagram illustrating a variation over time in the cuff pressure applied to the upper arm of the patient, and FIG. 5B is a diagram illustrating the pulse wave data extracted from the variation over time in the cuff pressure in FIG. 5A. FIG. 6 is a waveform diagram illustrating a waveform of the pulse wave for each step of the pulse wave data of FIG. 5B. FIG. 7 is a diagram illustrating a result of frequency analysis of the waveform of the pulse wave for each step illustrated in FIG. 6. FIG. 8 is a waveform diagram illustrating the waveform of the pulse wave for each step of the pulse wave including a respiratory component. FIG. 9 is a diagram illustrating a result of the frequency analysis of the pulse wave for each step illustrated in FIG. 8. FIG. 10 is a waveform diagram illustrating the waveform of the pulse wave including arrhythmia for each step. FIG. 11 is a diagram illustrating a result of the frequency analysis of the pulse wave for each step illustrated in FIG. 10. FIG. 12 is a schematic diagram illustrating a case where the measurement result of the central venous pressure and the information on the accuracy of the measurement result are displayed on a monitor screen.

In the present embodiment, a case where blood pressure measurement including an arterial pressure and a venous pressure of the patient is performed will be described. First, acquisition of a pulse wave of a patient is started (step S101). The pulse wave acquisition unit 401 controls the pulse wave measurer and starts acquiring the pulse wave of the patient (living body). The pulse wave acquisition unit 401 manages a timing at which the blood pressure measurement of the patient is started, and performs necessary control for each unit of the pulse wave measurer in accordance with the timing. For example, the pulse wave acquisition unit 401 may be configured to generate a trigger every predetermined period as the timing at which the blood pressure measurement is started. In addition, it is also possible to start the blood pressure measurement by using an instruction of the blood pressure measurement by the medical worker as the trigger.

When the timing at which the blood pressure measurement of the patient is performed or the instruction of the blood pressure measurement by the medical worker is input, the pulse wave acquisition unit 401 outputs an instruction to start measurement to the pulse wave measurer, and controls the pressurizing pump 311 and the exhaust valve 312. Accordingly, the pulse wave of the patient is acquired while the cuff pressure applied to the upper arm of the patient changes.

More specifically, as illustrated in FIG. 5A, when the blood pressure measurement is started, the pulse wave acquisition unit 401 causes the pressurizing pump 311 to increase the cuff pressure. In a process of increasing the cuff pressure (a period indicated by T1 in the figure), the arterial pressure, that is, a diastolic blood pressure, a mean blood pressure, and a systolic blood pressure are noninvasively measured by an oscillometric method. Since a method of measuring the arterial pressure by the oscillometric method is a known technique, a detailed description thereof is omitted.

Next, the central venous pressure is calculated (step S102). The pulse wave acquisition unit 401 lowers the cuff pressure, which increases to a pressure (for example, 130 mmHg or more) sufficient for the blood pressure measurement in step S101, to about 40 mmHg to 50 mmHg (for example, 45 mmHg), which is equal to or less than the diastolic blood pressure, and keeps the cuff pressure constant (a period indicated by T2 in the figure). Thereafter, the pulse wave acquisition unit 401 lowers the cuff pressure stepwise by a predetermined pressure reduction width (for example, 5 mmHg) every predetermined time (for example, 900 ms). The venous pressure calculation unit 402 calculates the central venous pressure of the patient based on the cuff pressure and a waveform of the pulse wave in a process of lowering the cuff pressure (a period indicated by T3 in the figure). A pressurizing section of each step at which the cuff pressure is lowered stepwise corresponds to each step in FIG. 6 described later. More specifically, a last pressurizing section of T2 and eleven pressurizing sections of T3 in FIG. 5A correspond to steps 1 to 12 of FIG. 6, respectively.

More specifically, as illustrated in FIG. 5B, the pulse wave is measured in the process of lowering the cuff pressure at a predetermined pressure reduction width. FIG. 5B illustrates variation over time in a component of pressure vibration extracted from a waveform showing variation over time of the cuff pressure illustrated in FIG. 5A. The component of the pressure vibration can be extracted by performing a filtering processing on the waveform illustrated in FIG. 5A. The filtering processing may be performed by passing a high-pass filter circuit that can be included in the cuff pressure detector 314, or may be performed by software by the one or more processors 361.

When the cuff pressure is lowered stepwise (step-shaped), a pulse wave component of an artery superimposed on the cuff pressure decreases accordingly, and a pulse wave component of a vein (hereinafter, also simply referred to as "pulse wave") gradually increases. When the cuff pressure is further lowered, the pulse wave further increases. Then, when the cuff pressure is equal to a pressure of the blood applied to a vascular wall of the upper arm of the patient, an amplitude of the pulse wave is maximized. The venous pressure calculation unit 402 determines that the cuff pressure at a time point when the amplitude of the pulse wave changes by a predetermined value, for example, at a time point when the amplitude of the pulse wave becomes maximum (the cuff pressure indicated by P1 in FIG. 5B) is an average venous pressure. The measurement of the pulse wave while lowering the cuff pressure is repeated until the amplitude of the pulse wave changes by the predetermined value. Then, the venous pressure calculation unit 402 estimates that a determined average venous pressure is the central venous pressure.

In examples of FIGS. 5A and 5B, a case is described in which the pulse wave is measured while the cuff pressure is lowered, and the venous pressure is estimated from the cuff pressure at which the amplitude of the pulse wave changes by the predetermined value, but the presently disclosed subject matter is not limited to such a case. The pulse wave may be measured while the cuff pressure is increased, and the venous pressure may be estimated from the cuff pressure at which the amplitude of the pulse wave changes by the predetermined value.

Thus, since the pulse wave analysis device 300 can measure the venous pressure of the patient by calculation based on the cuff pressure and the pulse wave, invasive measurement can be avoided.

In the present embodiment, in parallel with the measurement of the venous pressure, the vital parameter is also calculated by analyzing the pulse wave. More specifically, the parameter calculation unit 203 analyzes waveforms of the pulse wave in a predetermined period, and calculates at least one vital parameter for the patient. The predetermined period may be a process of lowering the cuff pressure (a period indicated by T3 in FIG. 5A). The predetermined period does not need to completely match the process of lowering the cuff pressure, and may be a period of a part of the process of lowering the cuff pressure or a period partially overlapping with the process of lowering the cuff pressure.

It is preferable that the parameter calculation unit 203 calculates the vital parameter while measuring the venous pressure in the process of lowering the cuff pressure. In the flow chart of FIG. 4, for convenience of description, it is described that the central venous pressure is calculated in step S102 and then the vital parameter is calculated in step S103, but processing of steps S102 and S103 can be performed simultaneously in parallel. Accordingly, it is possible to shorten a time required for the blood pressure measurement. In addition, after the venous pressure is measured, the waveform of the pulse wave in the predetermined period can be analyzed and the vital parameter can also be calculated.

Analysis of the pulse wave is performed, for example, by a frequency analysis (for example, Fourier transform) for the waveform of the pulse wave or calculation of a change in the amplitude. The vital parameter can include, for example, at least one of a respiration rate, a respiration depth, presence or absence of arrhythmia, a heart rate (pulse rate), and the arterial oxygen saturation (SpO2).

As illustrated in FIG. 6, each step (Steps 1 to 12 in an example illustrated in the figure) can include a plurality of pulse waves of one unit corresponding to one heart beat. In a normal pulse wave, variation of a cycle is little.

As illustrated in FIG. 7, the parameter calculation unit 203 performs the frequency analysis of the waveform of the pulse wave for each step of FIG. 6, and acquires an intensity of the pulse wave with respect to the frequency. In the figure, a vertical axis is the intensity (power display) of the pulse wave, and a horizontal axis is a frequency (displayed numerical value × 60) [Hz]. For example, when the heart beat of the patient is 60 times per minute (that is, 1 [Hz]), the horizontal axis has a peak (indicated by "O" in FIG. 7) at a frequency of 1 and a positive multiples (harmonics) thereof.

The heart beat of a healthy adult at rest is generally said to be about 60 times to 100 times per minute. The pulse wave measured by the pulse wave measurer may include not only a heart beat component derived from the heart beat of the patient but also the respiratory component derived from respiration. For example, as illustrated in FIG. 8, in the pulse wave including a large number of respiratory components, the respiratory component is superimposed on the heart beat component, so that the amplitude may vary.

The respiration rate is about 12 times to 20 times per minute, and is smaller than the heart rate. Therefore, since a cycle of the respiratory component included in the pulse wave usually has a longer cycle than a cycle of the heart beat component, a difference in a measured cycle of the pulse wave (that is, frequency) can be used to separate the respiratory component from the pulse wave. In addition, the intensity (power) of the respiratory component can be acquired from a size of the separated respiratory component.

More specifically, as illustrated in FIG. 9, the parameter calculation unit 203 performs the frequency analysis of the waveform of the pulse wave for each step of FIG. 8. In the figure, the vertical axis corresponds to the respiration depth, and the horizontal axis corresponds to the respiration rate per minute of the respiratory component of the pulse wave.

On the other hand, since the respiration of each patient is about 20 times per minute, the respiratory component of the pulse wave appears in a range of a frequency lower than 60 Hz. In an example illustrated in FIG. 9, the respiration appears at a frequency indicated by an arrow AR in each step. Therefore, for example, the parameter calculation unit 403 can separate the respiratory component from the pulse wave by a low-pass filter having a cut-off frequency of approximately 40 Hz. The parameter calculation unit 403 calculates the respiration depth in the pulse wave based on a peak value of the respiratory component. Accordingly, it can be estimated that when the respiration depth is deep, an influence of respiration on the pulse wave is large.

As a result of the frequency analysis of the pulse wave, since a component corresponding to a fundamental frequency is the heart beat component, the parameter calculation unit 403 calculates the heart rate (pulse rate) based on the fundamental frequency. Further, as illustrated in FIG. 10, when a pulse is disturbed by arrhythmia, variation occurs in the cycle of the pulse wave. In such a case, as illustrated in FIG. 11, when the frequency analysis is performed for each step, the peak appears at a frequency other than the harmonics of the fundamental frequency of the heart beat. The parameter calculation unit 403 can determine the presence or absence of arrhythmia in accordance with the fundamental frequency and the peak other than the harmonics. The parameter calculation unit 403 determines that there is arrhythmia, for example, when there is a peak greater than a predetermined value at a frequency other than the fundamental frequency and the harmonics, and determines that there is no arrhythmia when there is no peak greater than the predetermined value. Alternatively, instead of performing the frequency analysis, the parameter calculation unit 403 can also determine the presence or absence of arrhythmia in the pulse wave based on variation in the cycle (a time from a certain peak to a next peak) of the waveform of the pulse wave. For example, the parameter calculation unit 403 determines that there is arrhythmia when the variation of the cycle is large, and determines that there is no arrhythmia when the variation is small. In addition, for example, the parameter calculation unit 403 determines that there is arrhythmia when an average value of the cycle of the waveform of the pulse wave is longer than a predetermined first time, and also determines that there is arrhythmia when the average value of the cycle of the waveform of the pulse wave is shorter than a predetermined second time. Here, the predetermined second time is set shorter than the predetermined first time.

Next, the measurement result of the venous pressure and the information on the accuracy of the measurement result are output (Step S104). As illustrated in FIG. 12, the output controller 404 functions as an output, and performs control to display, for example, the measurement result of the venous pressure and the information on the accuracy of the measurement result (hereinafter, also referred to as "measurement accuracy information") on a monitor screen 500 of the display 341. In addition, the measurement result of the venous pressure and the measurement accuracy information may be output from the speaker 342 with a sound, or may be printed on a sheet when the output device 340 includes a printer. Alternately, the measurement result of the venous pressure and the measurement accuracy information may be transmitted to a server (computer) arranged on communication network. Alternately, the measurement result of the venous pressure and the measurement accuracy information may be provided online via a network such as the Internet.

The monitor screen 500 may include, for example, a venous pressure display region 510 for displaying the calculated central venous pressure (CVP), an accuracy display region 520 for displaying the measurement accuracy information, a heart rate display region 530 for displaying the heart rate (HR), a respiration information display region 540 for displaying the respiration rate (RR) and the respiration depth, a non-invasive blood pressure display region 550 for displaying a non-invasive blood pressure (NIBP), an oxygen saturation display region 560 for displaying the arterial oxygen saturation (SpO2), and a waveform display region 570 (waveform not illustrated).

FIG. 12 illustrates a case where the calculated central venous pressure is 20 mmHg and the measurement accuracy of the venous pressure is good. For example, the measurement accuracy information can include information on a level of the accuracy of the calculated venous pressure. When the accuracy is high, "GOOD" is displayed, and when the accuracy is low, "BAD" is displayed. For example, when the respiration depth is equal to or less than a predetermined threshold value (cut-off value) and there is no arrhythmia, the output controller 404 determines that the accuracy of the calculated venous pressure is high, and displays "GOOD" as the measurement accuracy. On the other hand, in at least one of a case where the respiration depth exceeds the above threshold value and a case where there is arrhythmia, the output controller 404 determines that the accuracy of the calculated venous pressure is low, and displays "BAD" as the measurement accuracy. Thus, according to a measurement result of the vital parameter (in the present example, a magnitude of the respiration depth or the presence or absence of arrhythmia), a magnitude of the measurement result and display of an index indicating a quality (for example, HIGH/LOW or GOOD/BAD) are added to display of the venous pressure. Therefore, the user can easily grasp the measurement accuracy of the venous pressure. Although a case where the measurement accuracy is displayed in two levels of "GOOD" and "BAD" is described, the presently disclosed subject matter is not limited thereto, and the accuracy may be displayed in multiple levels by setting a plurality of threshold values. In addition to textual indications (for example, HIGH/LOW or GOOD/BAD), indications using marks such as **★★★★/★★★☆** may also be used for displaying a magnitude of the measurement result and display of an index indicating a quality.

The heart rate (HR), the respiration rate (RR), respiration information, the noninvasive blood pressure (NIBP), and the arterial oxygen saturation (SpO2) are displayed on the monitor screen 500 in a normal physiological information measurement, but display is omitted here in order to clarify a difference from Modification to be described later.

The output controller 404 can also control the output of the calculated venous pressure based on at least one of the vital parameter and the measurement accuracy information. For example, the output controller 404 can change, based on at least one of the vital parameter and the measurement accuracy information, at least one of the display/non-display of the measurement result of the venous pressure, a display color and/or a display size of the measurement result, and presence or absence of a warning display related to the measurement accuracy. For example, the output controller 404 can control to display the measurement result when the measurement accuracy is high, and not to display the measurement result when the measurement accuracy is low.

In addition, the output controller 404 can display measurement results in different colors according to the level of the measurement accuracy. For example, the output device 340 can be controlled so that the display color of the measurement result is displayed in green when the measurement accuracy is high, and the display color of the measurement result is displayed in red when the measurement accuracy is low.

In addition, the output controller 404 can control the output device 340 so as to display the measurement results in different sizes according to the level of the measurement accuracy. For example, the output controller 404 can control the output device 340 so that the display size of the measurement result is a normal size when the measurement accuracy is high, and the display size of the measurement result is larger than the normal size when the measurement accuracy is low.

In addition, when the measurement accuracy is low, the measurement result can be marked or surrounded by a frame to warn that the measurement accuracy is low. Thus, since output forms of the venous pressure are different according to a level of calculated measurement accuracy of the venous pressure, the user can easily grasp that the measurement accuracy is low.

In addition, when the calculation result of the vital parameter is not included in an appropriate range (predetermined range) of the vital parameter, the output controller 404 can also control the output device 340 to output an alarm by display and/or sound. Accordingly, the user can instantly grasp that the calculation result of the vital parameter is out of the appropriate range of the vital parameter.

Further, when the measurement result of the venous pressure is not included in an appropriate range (predetermined range) of the venous pressure, the output controller 404 can also control the output device 340 to output an alarm by display and/or sound. Accordingly, the user can instantly grasp that the calculation result of the venous pressure is out of the appropriate range of the venous pressure.

In a processing of the flow chart illustrated in FIG. 4 described above, the pulse wave acquisition unit 401 acquires the pulse wave of the patient. The venous pressure calculation unit 402 calculates the venous pressure based on the waveform of the pulse wave in the predetermined period, and the parameter calculation unit 403 analyzes the waveform in the predetermined period and calculates at least one vital parameter for the patient. Then, the output controller 404 outputs the calculated venous pressure and the information on the accuracy of the venous pressure based on the vital parameter.

### (Modification)

In the above example, a case where the measurement result of the central venous pressure and the information on the accuracy of the measurement result are displayed on the monitor screen is described, but it is also possible to adopt a configuration in which the vital parameter is output instead of the information on the accuracy of the measurement result or in addition to the information on the accuracy of the measurement result.

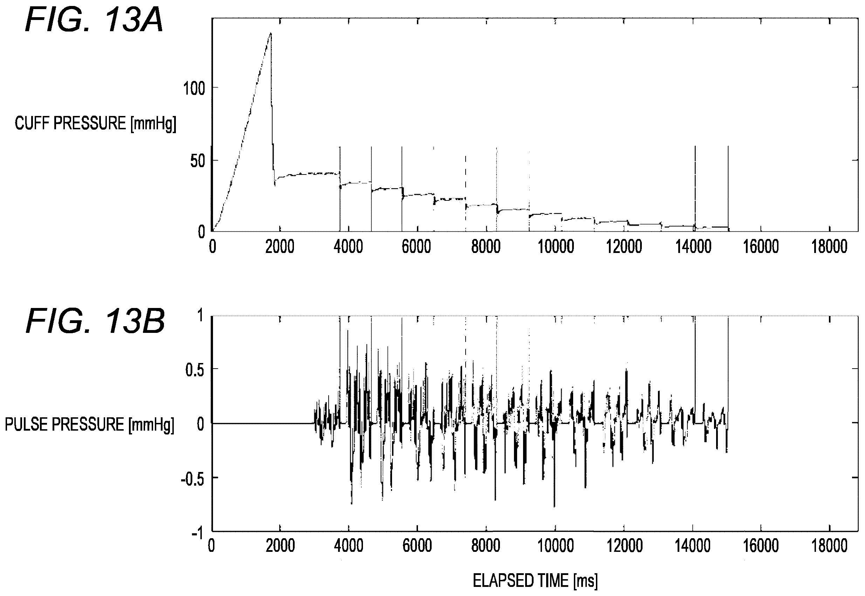

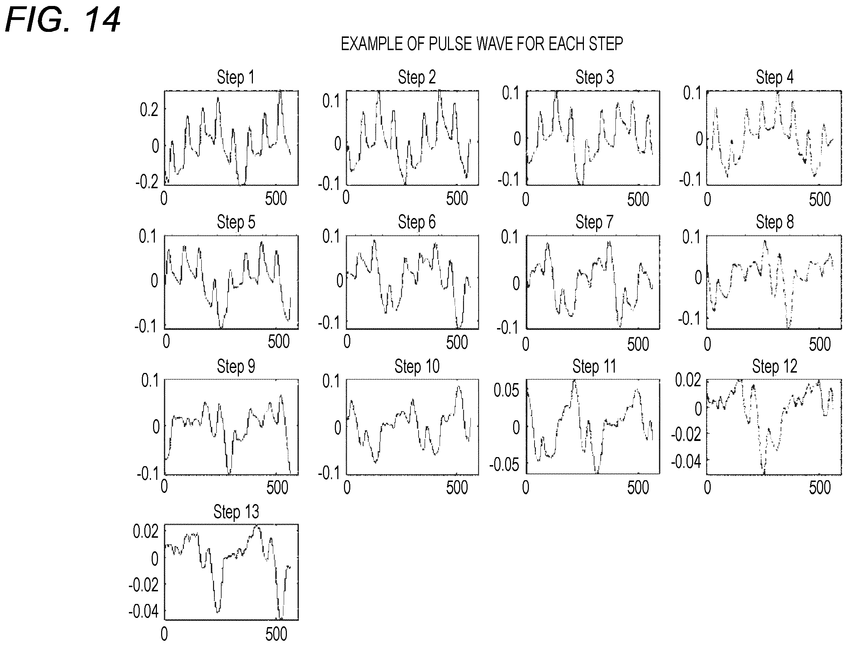

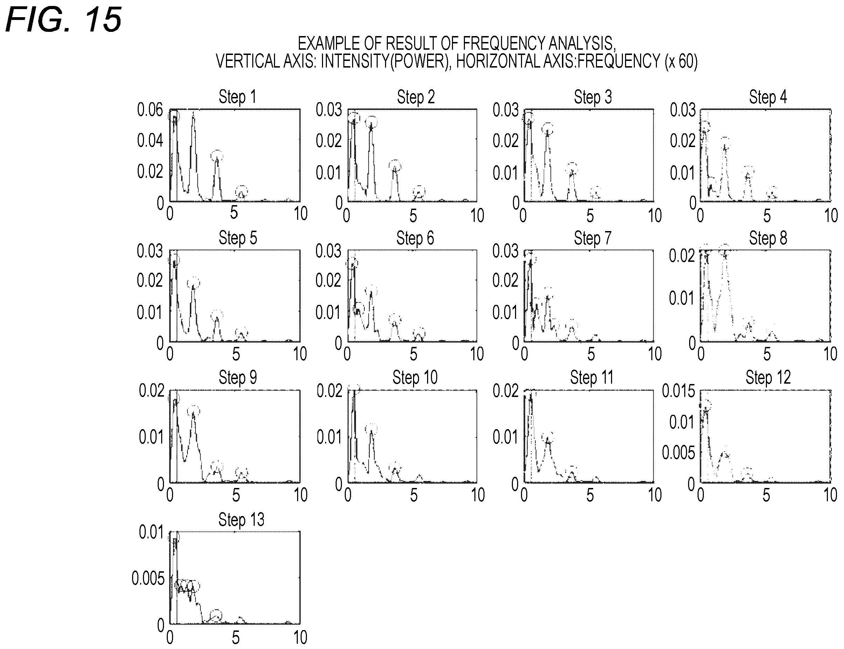

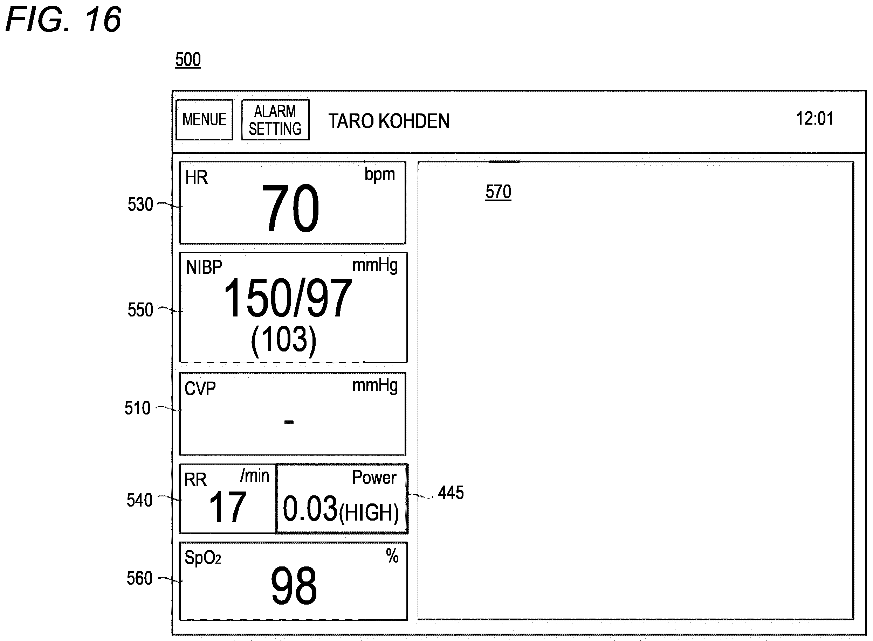

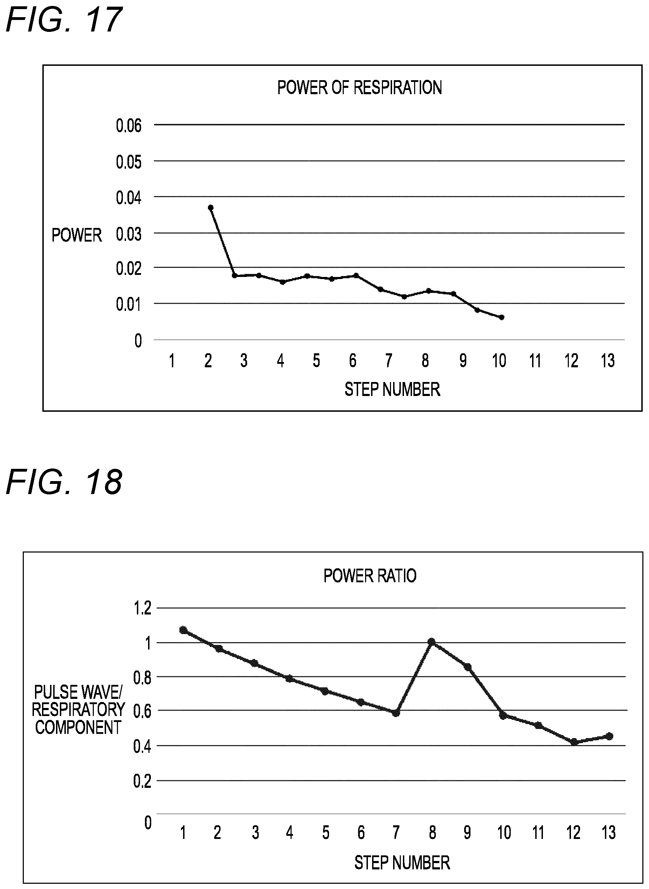

It is assumed that the respiratory component contained in the pulse wave is large. FIG. 13A is a diagram illustrating the variation over time in the cuff pressure applied to the upper arm of the patient, and FIG. 13B is a diagram illustrating the pulse wave data extracted from the variation over time in the cuff pressure in FIG. 13A. FIG. 14 is a waveform diagram illustrating the waveform of the pulse wave for each step of the pulse wave data illustrated in FIG. 13B. FIG. 15 is a diagram illustrating the result of the frequency analysis of the pulse wave for each step illustrated in FIG. 14. FIG. 16 is a schematic diagram illustrating a case where the measurement result of the central venous pressure and the respiration depth as the vital parameter are displayed on the monitor screen. FIG. 17 is a graph illustrating power of respiration for each step. FIG. 18 is a graph illustrating the heart beat component/the respiratory component for each step.

As illustrated in FIGS. 13A and 13B, the pulse wave acquisition unit 401 acquires the pulse wave of the patient while changing the cuff pressure applied to the upper arm of the patient. However, in examples illustrated in the figure, since the amplitude of the pulse wave significantly fluctuates due to an influence of respiration in an entire lowering process of the cuff pressure, it is difficult to determine the time point when the amplitude of the pulse wave becomes maximum, and it is not possible to determine the venous pressure. As illustrated in FIG. 14, the amplitude of the pulse wave for each step also varies for each unit of the pulse wave.

As illustrated in FIG. 15, the parameter calculation unit 403 performs the frequency analysis of the pulse wave for each step. As a result of the analysis, in many steps, the peak appears in a low frequency region where the frequency is equal to or less than 1 [Hz], and the respiratory component is larger than the heart beat component. For example, the intensity (respiration depth) of the respiratory component is about 0.06 in Step 1, and about 0.03 in Step 2.

As illustrated in FIG. 16, the output controller 404 controls the output device 340 to display the measurement result of the central venous pressure and the respiration depth as the vital parameter in the venous pressure display region 510 and the respiration information display region 540, respectively. The figure illustrates a case where the respiration rate is 17 [/min] and the respiration depth (Power) is 0.03. The central venous pressure cannot be measured due to the influence of respiration, so the central venous pressure is displayed as "-". In addition, the output controller 404 can perform control so as to compare a value of the respiration depth with the cut-off value of the respiration depth and display an index (for example, high or low) indicating the magnitude of the respiration depth according to a comparison result. For example, in the figure, since the value of the respiration depth is larger than the cut-off value (for example, 0.01), "HIGH" is displayed as a level of the respiration depth. Thus, a high/low display is added to display of the respiration depth according to the magnitude of the respiration depth as the calculation result as the vital parameter. Therefore, the user can easily grasp the level of the respiration depth. The cut-off value is stored in the RAM of the controller 360 in advance. In the example described above, a case where two levels of high/low levels are displayed for the respiration depth is described, but the presently disclosed subject matter is not limited to two-level display, and a plurality of threshold values for comparison may be stored in the RAM so as to display multiple levels (for example, high/middle/low). The doctor confirms that the respiration depth is high (deep), and determines that the central venous pressure cannot be measured because the measurement of the central venous pressure is affected by respiration.

Thus, in a case where a baseline of the pulse wave is not stabilized due to the influence of respiration and it is difficult to detect an amplitude change of the pulse wave, a processing is performed to display that the accuracy of the venous pressure is low or to hide the measurement result. Accordingly, the user can grasp that the influence of respiration is large, the accuracy of the venous pressure is low, or the measurement cannot be performed.

In addition, it is also possible to calculate the venous pressure from the fluctuation in a magnitude of the pulse wave (heart beat component), instead of the amplitude change of the pulse wave. For example, as illustrated in FIG. 17, when it is difficult to detect an amplitude change of the heart beat component because the respiratory component is superimposed, the venous pressure is estimated using the fluctuation of the magnitude of the pulse wave instead of the amplitude change of the heart beat component. For example, in Steps 2 to 11 of the figure, when the respiration depth of the patient is substantially constant, that is, power of the respiratory component is substantially constant, if power of the heart beat component is also constant, a ratio of power of the pulse wave to the power of the respiratory component is substantially constant. However, as illustrated in FIG. 18, although the respiration depth is substantially constant in Steps 2 to 11, the ratio of the power of the pulse wave to the power of the respiration component greatly changes. This is because an amplitude of the heart beat component changes. In an example illustrated in FIG. 18, the venous pressure calculation unit 402 determines that the cuff pressure corresponding to Step 8 in which the ratio of the power of the pulse wave to the power of the respiratory component is the largest is the central venous pressure.

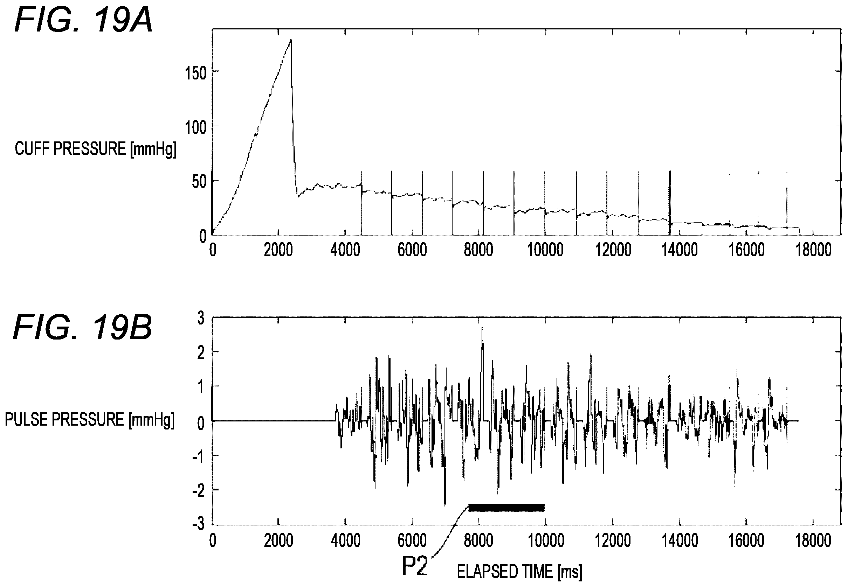

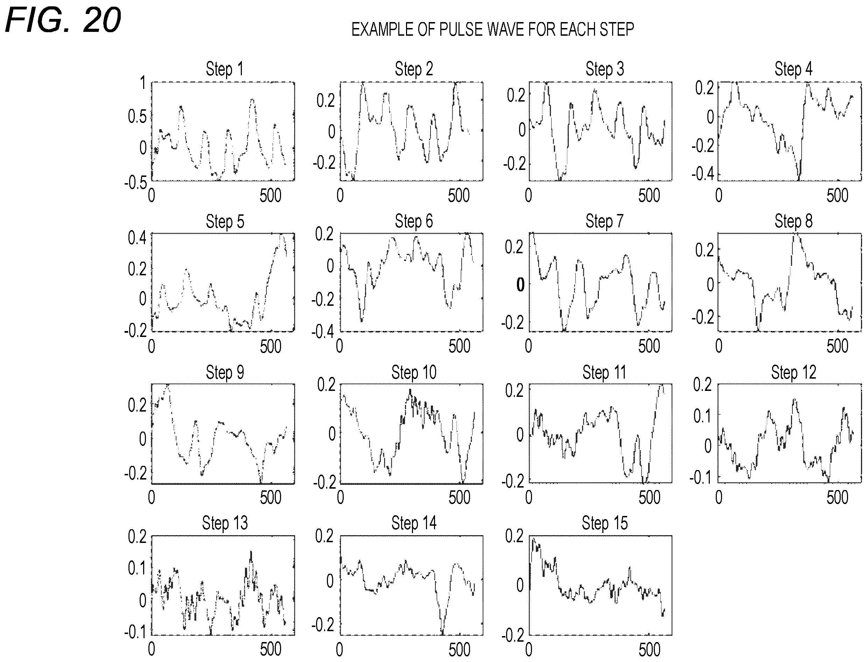

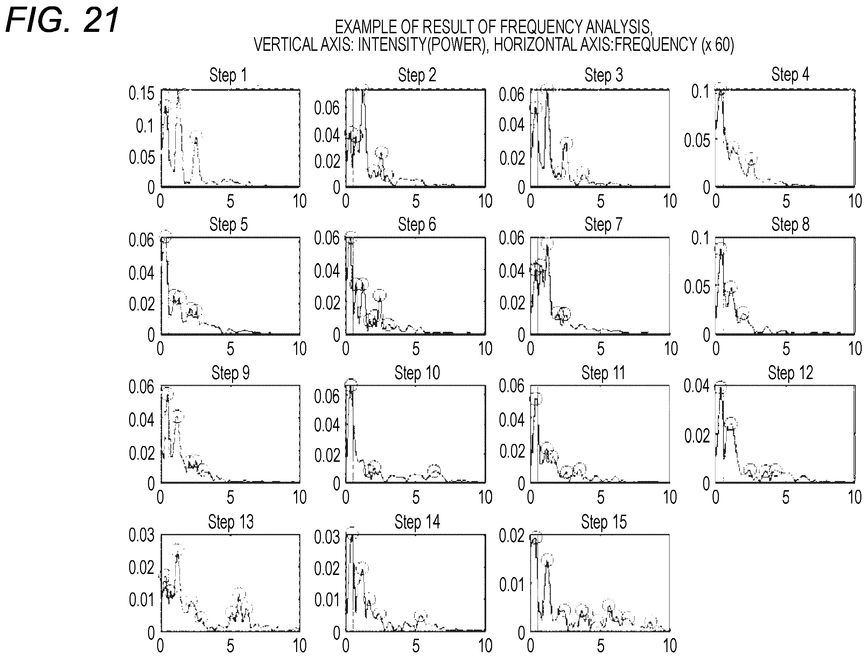

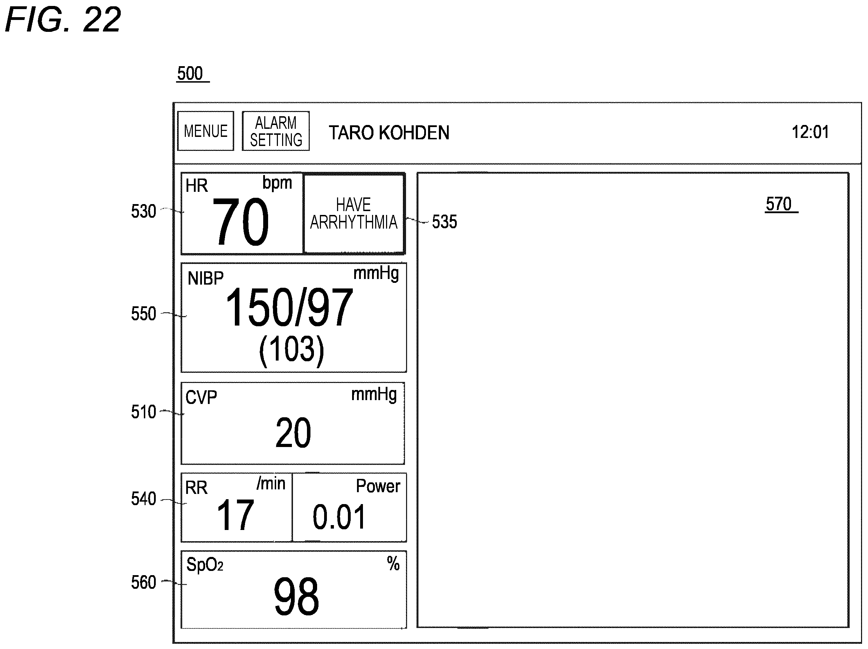

In addition, it is assumed that the cycle of the heart beat component of the pulse wave varies. FIG. 19A is a diagram illustrating the variation over time in the cuff pressure applied to the upper arm of the patient, and FIG. 19B is a diagram illustrating the pulse wave data extracted from the variation over time in the cuff pressure in FIG. 19A. FIG. 20 is a waveform diagram illustrating the waveform of the pulse wave for each step of the pulse wave data illustrated in FIG. 19B. FIG. 21 is a diagram illustrating the result of the frequency analysis of the pulse wave for each step illustrated in FIG. 20. FIG. 22 is a schematic diagram illustrating a case where the measurement result of the central venous pressure and presence or absence of arrhythmia as the vital parameter are displayed on the monitor screen.

As illustrated in FIGS. 19A and 19B, the pulse wave acquisition unit 401 acquires the pulse wave of the patient while changing the cuff pressure applied to the upper arm of the patient. The venous pressure calculation unit 402 determines that the cuff pressure at the time point when the amplitude of the pulse wave becomes maximum (the cuff pressure indicated by P2 in FIG. 19B) is the central venous pressure. As illustrated in FIG. 20, the cycle varies in the pulse wave for each step.

As illustrated in FIG. 21, the parameter calculation unit 403 performs the frequency analysis of the pulse wave for each step in FIG. 20. As a result of the analysis, a peak value appears also at a frequency other than harmonics of the heart beat component. This is because the cycle of the heart beat component of the pulse wave varies. Therefore, it is considered that arrhythmia is included in the pulse wave, and the accuracy of the measurement result of the central venous pressure calculated by the venous pressure calculation unit 402 is low.

As illustrated in FIG. 22, the output controller 404 controls the output device 340 to display the measurement result of the central venous pressure and the presence or absence of arrhythmia as the vital parameter in the venous pressure display region 510 and the arrhythmia display region 535, respectively. The figure illustrates a case where the central venous pressure is 20 mmHg and there is arrhythmia. The doctor confirms that the patient has arrhythmia and determines that the accuracy of the measurement result is low because the measurement of central venous pressure is affected by arrhythmia.

The pulse wave analysis device of the present embodiment described above achieves following effects.

Since the measured central venous pressure, the vital parameter, and/or the information on the accuracy of the central venous pressure based on the vital parameter are output, the central venous pressure of the patient and the accuracy of the measured central venous pressure can be notified to the medical worker. Therefore, the medical worker can confirm the measured central venous pressure after recognizing the measurement accuracy.

In addition, since the measurement of the venous pressure and the calculation of the vital parameter can be performed simultaneously in parallel, a measurement time can be shortened.

As described above, in the embodiment, the pulse wave analysis device, the pulse wave analysis method, and the pulse wave analysis program of the presently disclosed subject matter are described. However, it is needless to say that the presently disclosed subject matter can be appropriately added, modified, and omitted by those skilled in the art within the scope of the technical idea.

For example, in the above embodiment, a case where the pulse wave analysis device 300 performs the blood pressure measurement including the arterial pressure and the venous pressure of the patient is described, but the presently disclosed subject matter is not limited to such a case. After starting the measurement, the pulse wave analysis device 300 may measure the central venous pressure by lowering or increasing the cuff pressure stepwise without measuring the arterial pressure. In addition, the arterial pressure may be measured after the venous pressure is measured.

In the above embodiment, a case where the venous pressure is calculated based on the pulse wave (cuff pulse wave) measured by the pulse wave measurer including the cuff 200 is mainly described. However, the presently disclosed subject matter is not limited to such a case. For example, the venous pressure may be calculated based on the photoplethysmogram measured by the photoplethysmogram measurer. The venous pressure may be calculated based on the pulse wave data measured noninvasively by another system and acquired from a server (computer) arranged on communication network via the network interface 350. In addition, a sensor with a built-in light source and photodetector may be disposed in a neck of the patient, and the venous pressure may be estimated by performing an operation using near infrared spectroscopy (NIRS) on an optical signal acquired by the sensor.

In addition, in the above embodiment, a case is described in which the measurement result of the venous pressure or the calculation result of the vital parameter is independently compared with a threshold value, or determination as to whether the measurement result of the venous pressure and the calculation result of the vital parameter are included in the appropriate range is performed, the measurement accuracy is displayed, and the alarm is output, but the presently disclosed subject matter is not limited to such a case. In consideration of the measurement result of the venous pressure and the influence of each of the at least one vital parameter on another measurement (calculation), the measurement accuracy (reliability) may be displayed, and the alarm may be output. In addition, a configuration may be adopted in which a new index is generated by combining a plurality of vital parameters, a comparison with the threshold value is performed, the measurement accuracy is displayed, and the alarm is output.

In order to improve the measurement accuracy of the pulse wave, the photoplethysmogram may be added. In addition, an electrode and an electrocardiogram measuring device may be further included and a pulse wave by an electrocardiogram may be added.

A section and method of performing various processing in the pulse wave analysis device 300 according to the above embodiment can be implemented by either a dedicated hardware circuit or a programmed computer. The program may be provided by a computer-readable recording medium such as a compact disc read only memory (CD-ROM), or may be provided online via a network such as the Internet. In this case, the program recorded in the computer-readable recording medium is usually transferred to and stored in a memory such as a hard disk. The program may be provided as independent application software, or may be incorporated into software of the pulse wave analysis device 300 as one function of the pulse wave analysis device 300.

## Claims

1. A pulse wave analysis device (300) comprising:
a pulse wave acquisition unit (401) configured to acquire a pulse wave of a living body;
a venous pressure calculation unit (402) configured to calculate a venous pressure based on a waveform of the pulse wave in a predetermined period;
a parameter calculation unit (403) configured to calculate at least one vital parameter for the living body by analyzing the waveform of the pulse wave in the predetermined period; and
an output (404) configured to output the calculated venous pressure, the calculated vital parameter, and information on the accuracy of the venous pressure, said information being determined based on the vital parameter

2. The pulse wave analysis device (300) according to claim 1,
wherein the pulse wave acquisition unit (401) is connected to a cuff (200) worn on the living body, acquires a pulse wave of the living body while changing a cuff pressure applied to the living body, and
wherein the venous pressure calculation (402) unit calculates the venous pressure based on the waveform and the cuff pressure.

3. The pulse wave analysis device (300) according to claim 1 or 2, wherein the vital parameter includes at least one of a respiration rate, a respiration depth, presence or absence of arrhythmia, a pulse rate, and arterial oxygen saturation.

4. The pulse wave analysis device (300) according to claim 1 or 2,
wherein the vital parameter includes at least one of a respiration rate, a respiration depth, and presence or absence of arrhythmia, and
wherein the parameter calculation unit (403) performs frequency analysis of the waveform and calculates at least one of the respiration rate, the respiration depth, and the presence or absence of arrhythmia in the pulse wave.

5. The pulse wave analysis device (300) according to claim 1 or 2,
wherein the vital parameter includes presence or absence of arrhythmia, and
wherein the parameter calculation unit (403) determines the presence or absence of arrhythmia in the pulse wave based on variation in a cycle of the waveform.

6. The pulse wave analysis device (300) according to claim 1 or 2,
wherein the vital parameter includes a respiration depth, and
wherein the parameter calculation unit (403) calculates the respiration depth in the pulse wave based on a peak value obtained when frequency analysis of the waveform is performed.

7. The pulse wave analysis device (300) according to any one of claims 1 to 6, wherein based on at least one of the vital parameter and the information on the accuracy of the venous pressure, the output (404) controls output of the calculated venous pressure.

8. The pulse wave analysis device (300) according to claim 7, wherein the output changes, based on at least one of the vital parameter and the information on the accuracy of the venous pressure, at least one of display/non-display of the venous pressure, a display color and/or a display size of the venous pressure, and presence or absence of a warning display related to the accuracy of the venous pressure.

9. The pulse wave analysis device (300) according to any one of claims 1 to 8, further comprising:
a first memory (362) configured to store a cut-off value for the venous pressure and a cut-off value for the vital parameter,
wherein the output (404) includes a display (341) that displays the venous pressure and the vital parameter, and
wherein a value of the venous pressure and a value of the vital parameter are compared with the cut-off value of the venous pressure and the cut-off value of the vital parameter, respectively, and displays of the venous pressure and/or the vital parameter are made different according to comparison results.

10. The pulse wave analysis device (300) according to claim 9, wherein the output (404) makes a display color and/or a display size of the venous pressure and/or the vital parameter different in accordance with a magnitude of the value of the venous pressure and the value of the vital parameter.

11. The pulse wave analysis device (300) according to any one of claims 1 to 9,
wherein the output (404) includes the display (341) that displays the venous pressure and the vital parameter, and
wherein in accordance with a magnitude of the value of the venous pressure and the value of the vital parameter, display of an index indicating the magnitude is added to display of the venous pressure and/or the vital parameter.

12. The pulse wave analysis device (300) according to any one of claims 1 to 11, further comprising:
a second memory (362) configured to store a predetermined range for the venous pressure and the vital parameter,
wherein the output (404) outputs an alarm when the value of the venous pressure and the value of the vital parameter are not included in the predetermined range.

13. A computer-implemented pulse wave analysis method comprising:
acquiring a pulse wave of a living body;
calculating a venous pressure based on a waveform of the pulse wave in a predetermined period;
calculating at least one vital parameter for the living body by analyzing the waveform of the pulse wave in the predetermined period; and
outputting the calculated venous pressure, the calculated vital parameter, and information or the accuracy of the venous pressure, said information being determined based on the vital parameter.

14. A pulse wave analysis program that, when executed by a computer with a pulse wave acquisition unit, causes it to execute a process including:
acquiring a pulse wave of a living body;
calculating a venous pressure based on a waveform of the pulse wave in a predetermined period;
calculating at least one vital parameter for the living body by analyzing the waveform of the pulse wave in the predetermined period; and
outputting the calculated venous pressure, the calculated vital parameter, and information on the accuracy of the venous pressure, said information being determined based on the vital parameter.

## Patentansprüche

1. Pulswellen-Analysevorrichtung (300), die umfasst:
eine Pulswellen-Erfassungseinheit (401), die zum Erfassen einer Pulswelle eines lebenden Körpers ausgeführt ist;
eine Venendruck-Berechnungseinheit (402), die so ausgeführt ist, dass sie einen Venendruck auf Basis einer Wellenform der Pulswelle in einer vorgegebenen Zeitspanne berechnet;
eine Parameter-Berechnungseinheit (403), die so ausgeführt ist, dass sie wenigstens einen Vitalparameter für den lebenden Körper berechnet, indem sie die Wellenform der Pulswelle in der vorgegebenen Zeitspanne analysiert; sowie
einen Ausgang (404), der so ausgeführt ist, dass er den berechneten Venendruck, den berechneten Vitalparameter sowie eine Information über die Genauigkeit des Venendrucks ausgibt, wobei die Information auf Basis des Vitalparameters bestimmt wird.

2. Pulswellen-Analysevorrichtung (300) nach Anspruch 1,
wobei die Pulswellen-Erfassungseinheit (401) mit einer an dem lebenden körpergetragenen Manschette (200) verbunden ist, eine Pulswelle des lebenden Körpers beim Ändern eines auf den lebenden Körper ausgeübten Manschettendrucks erfasst, und
wobei die Venendruck-Berechnungseinheit (402) den Venendruck auf Basis der Wellenform sowie des Manschettendrucks berechnet.

3. Pulswellen-Analysevorrichtung (300) nach Anspruch 1 oder 2, wobei der Vitalparameter
wenigstens eine Atemfrequenz, eine Atemtiefe, Vorhandensein oder Fehlen von Arrhythmie, eine Pulsfrequenz oder eine arterielle Sauerstoffsättigung umfasst.

4. Pulswellen-Analysevorrichtung (300) nach Anspruch 1 oder 2,
wobei der Vitalparameter wenigstens eine Atemfrequenz, eine Atemtiefe, sowie Vorhandensein oder Fehlen von Arrhythmie umfasst, und
wobei die Parameter-Berechnungseinheit (403) Frequenzanalyse der Wellenform durchführt und wenigstens die Atemfrequenz, die Atemtiefe, sowie das Vorhandensein oder Fehlen von Arrhythmie in der Pulswelle berechnet.

5. Pulswellen-Analysevorrichtung (300) nach Anspruch 1 oder 2,
wobei der Vitalparameter Vorhandensein oder Fehlen von Arrhythmie einschließt und wobei die Parameter-Berechnungseinheit (403) das Vorhandensein oder Fehlen von Arrhythmie in der Pulswelle auf Basis von Änderung in einem Zyklus der Wellenform bestimmt.

6. Pulswellen-Analysevorrichtung (300) nach Anspruch 1 oder 2,
wobei der Vitalparameter eine Atemtiefe einschließt, und
wobei die Parameter-Berechnungs-Einheit (403) die Atemtiefe in der Pulswelle auf Basis eines Spitzenwertes berechnet, der beim Durchführen von Frequenzanalyse der Wellenform ermittelt wird.

7. Pulswellen-Analysevorrichtung (300) nach einem der Ansprüche 1 bis 6, wobei der Ausgang (404) Ausgabe des berechneten Venendrucks auf Basis wenigstens des Vitalparameters oder der Information über die Genauigkeit des Venendrucks steuert.

8. Pulswellen-Analysevorrichtung (300) nach Anspruch 7, wobei sich die Ausgabe auf Basis wenigstens des Vitalparameters oder der Information über die Genauigkeit des Venendrucks, wenigstens Anzeige/Nichtanzeige des Venendrucks, einer Anzeigefarbe und/oder einer Anzeigegröße des Venendrucks sowie Vorhandensein oder Fehlen einer Warnanzeige ändert, die sich auf die Genauigkeit des Venendrucks bezieht.

9. Pulswellen-Analysevorrichtung (300) nach einem der Ansprüche 1 bis 8, die des Weiteren umfasst:
einen ersten Speicher (362),der so ausgeführt ist, dass er einen Grenzwert für den Venendruck und einen Grenzwert für den Vitalparameter speichert,
wobei der Ausgang (404) eine Anzeige (341) aufweist, die den Venendruck und den Vitalparameter anzeigt, und
wobei ein Wert des Venendrucks sowie ein Wert des Vitalparameters mit dem Grenzwert des Venendrucks bzw. dem Grenzwert des Vitalparameters verglichen werden und Anzeigen des Venendrucks und/oder des Vitalparameters gemäß den Vergleichsergebnissen geändert werden.

10. Pulswellen-Analysevorrichtung (300) nach Anspruch 9, wobei der Ausgang (404) eine Anzeigefarbe und/oder eine Anzeigegröße des Venendrucks und/oder des Vitalparameters entsprechend einem Betrag des Wertes des Venendrucks sowie des Wertes des Vitalparameters ändert.

11. Pulswellen-Analysevorrichtung (300) nach einem der Ansprüche 1 bis 9,
wobei der Ausgang (404) die Anzeige (341) aufweist, die den Venendruck sowie den Vitalparameter anzeigt, und
wobei entsprechend einem Betrag des Wertes des Venendrucks sowie des Wertes des Vitalparameters Anzeige eines Index, der den Betrag angibt, zu Anzeige des Venendrucks und/oder des Vitalparameters hinzugefügt wird.

12. Pulswellen-Analysevorrichtung (300) nach einem der Ansprüche 1 bis 11, die des Weiteren umfasst:
einen zweiten Speicher (362),der so ausgeführt ist, dass er einen vorgegebenen Bereich für den Venendruck und den Vitalparameter speichert,
wobei der Ausgang (404) eine Warnmeldung ausgibt, wenn der Wert des Venendrucks und der Wert des Vitalparameters nicht in dem vorgegebenen Bereich enthalten sind.

13. Computerimplementiertes Pulswellen-Analyseverfahren, das umfasst:
Erfassen einer Pulswelle eines lebenden Körpers;
Berechnen eines Venendrucks auf Basis einer Wellenform der Pulswelle in einer vorgegebenen Zeitspanne;
Berechnen wenigstens eines Vitalparameters für den lebenden Körper durch Analysieren der Wellenform der Pulswelle in der vorgegebenen Zeitspanne; sowie
Ausgeben des berechneten Venendrucks, des berechneten Vitalparameters sowie einer Information über die Genauigkeit des Venendrucks, wobei die Information auf Basis des Vitalparameters bestimmt wird.

14. Pulswellen-Analyseprogramm, das, wenn es von einem Computer mit einer Pulswellen-Erfassungseinheit ausgeführt wird, ihn veranlasst, einen Prozess auszuführen, der einschließt:
Erfassen einer Pulswelle eines lebenden Körpers;
Berechnen eines Venendrucks auf Basis einer Wellenform der Pulswelle in einer vorgegebenen Zeitspanne;
Berechnen wenigstens eines Vitalparameters für den lebenden Körper durch Analysieren der Wellenform der Pulswelle in der vorgegebenen Zeitspanne; sowie
Ausgeben des berechneten Venendrucks, des berechneten Vitalparameters sowie einer Information über die Genauigkeit des Venendrucks, wobei die Information auf Basis des Vitalparameters bestimmt wird.

## Revendications

1. Dispositif d'analyse d'onde de pouls (300), comprenant :
une unité d'acquisition d'ondes de pouls (401) configurée pour acquérir une onde de pouls d'un corps vivant ;
une unité de calcul de pression veineuse (402) configurée pour calculer une pression veineuse sur la base d'une forme d'onde de l'onde de pouls dans une période prédéterminée ;
une unité de calcul de paramètres (403) configurée pour calculer au moins un paramètre vital pour le corps vivant en analysant la forme d'onde de l'onde de pouls dans la période prédéterminée ; et
une sortie (404) configurée pour sortir la pression veineuse calculée, le paramètre vital calculé et de l'information sur la précision de la pression veineuse, ladite information étant déterminée sur la base du paramètre vital.

2. Dispositif d'analyse d'onde de pouls (300) selon la revendication 1,
dans lequel l'unité d'acquisition d'ondes de pouls (401) est connectée à un brassard (200) porté sur le corps vivant, et acquiert une onde de pouls du corps vivant tout en modifiant la pression du brassard appliquée au corps vivant, et
dans lequel l'unité de calcul de pression veineuse (402) calcule la pression veineuse sur la base de la forme d'onde et de la pression du brassard.

3. Dispositif d'analyse d'onde de pouls (300) selon la revendication 1 ou 2, dans lequel le paramètre vital inclut au moins un paramètre parmi la fréquence respiratoire, l'amplitude respiratoire, la présence ou l'absence d'arythmie, le rythme cardiaque, et la saturation artérielle en oxygène.

4. Dispositif d'analyse d'onde de pouls (300) selon la revendication 1 ou 2,
dans lequel le paramètre vital inclut au moins un paramètre parmi la fréquence respiratoire, l'amplitude respiratoire, et la présence ou l'absence d'arythmie, et
dans lequel l'unité de calcul de paramètres (403) met en œuvre une analyse de fréquence de la forme d'onde et calcule au moins un paramètre parmi la fréquence respiratoire, l'amplitude respiratoire, et la présence ou l'absence d'arythmie dans l'onde de pouls.

5. Dispositif d'analyse d'onde de pouls (300) selon la revendication 1 ou 2,
dans lequel le paramètre vital inclut la présence ou l'absence d'arythmie, et
dans lequel l'unité de calcul de paramètres (403) détermine la présence ou l'absence d'arythmie dans l'onde de pouls sur la base des variations de cycle de la forme d'onde.

6. Dispositif d'analyse d'onde de pouls (300) selon la revendication 1 ou 2,
dans lequel le paramètre vital inclut l'amplitude respiratoire, et
dans lequel l'unité de calcul de paramètres (403) calcule l'amplitude respiratoire dans l'onde de pouls sur la base d'une valeur de crête obtenue lors de la mise en œuvre d'une analyse de fréquence de la forme d'onde.

7. Dispositif d'analyse d'onde de pouls (300) selon l'une quelconque des revendications 1 à 6, dans lequel, sur la base d'au moins un élément parmi le paramètre vital et l'information sur la précision de la pression veineuse, la sortie (404) contrôle la sortie de la pression veineuse calculée.

8. Dispositif d'analyse d'onde de pouls (300) selon la revendication 7, dans lequel la sortie change, sur la base d'au moins un élément parmi le paramètre vital et l'information sur la précision de la pression veineuse, au moins paramètre parmi l'affichage/non-affichage de la pression veineuse, la couleur d'affichage et/ou la taille d'affichage de la pression veineuse, et la présence ou l'absence d'un affichage d'avertissement relatif à la précision de la pression veineuse.

9. Dispositif d'analyse d'onde de pouls (300) selon l'une quelconque des revendications 1 à 8, comprenant en outre :
une première mémoire (362) configurée pour stocker une valeur seuil pour la pression veineuse et une valeur seuil pour le paramètre vital,
dans lequel la sortie (404) inclut un affichage (341) qui affiche la pression veineuse et le paramètre vital, et
dans lequel une valeur de la pression veineuse et une valeur du paramètre vital sont comparées respectivement à la valeur seuil de la pression veineuse et à la valeur seuil du paramètre vital, et l'affichage de la pression veineuse et/ou des paramètres vitaux est adapté en fonction des résultats de comparaison.

10. Dispositif d'analyse d'onde de pouls (300) selon la revendication 9, dans lequel la sortie (404) commande la variation d'une couleur d'affichage et/ou d'une taille d'affichage de la pression veineuse et/ou du paramètre vital en fonction de la grandeur de la valeur de la pression veineuse et de la valeur du paramètre vital.

11. Dispositif d'analyse d'onde de pouls (300) selon l'une quelconque des revendications 1 à 9,
dans lequel la sortie (404) inclut l'affichage par l'affichage (341) de la pression veineuse et du paramètre vital, et
dans lequel, en fonction de la grandeur de la valeur de la pression veineuse et de la valeur du paramètre vital, l'affichage d'un indice indiquant la grandeur est ajouté à l'affichage de la pression veineuse et/ou du paramètre vital.

12. Dispositif d'analyse d'onde de pouls (300) selon l'une quelconque des revendications 1 à 11, comprenant en outre :
une deuxième mémoire (362) configurée pour stocker une plage prédéterminée pour la pression veineuse et le paramètre vital,
dans lequel la sortie (404) déclenche une alarme quand la valeur de la pression veineuse et la valeur du paramètre vital ne sont pas incluses dans la plage prédéterminée.

13. Procédé d'analyse d'onde de pouls implémenté par un ordinateur, comprenant :
l'acquisition d'une onde de pouls d'un corps vivant ;
le calcul d'une pression veineuse sur la base de la forme d'onde de l'onde de pouls sur une période prédéterminée ;
le calcul d'au moins un paramètre vital pour le corps vivant en analysant la forme d'onde de l'onde de pouls dans la période prédéterminée ; et
la sortie de la pression veineuse calculée, du paramètre vital calculé et de l'information sur la précision de la pression veineuse, ladite information étant déterminée sur la base du paramètre vital.

14. Programme d'analyse d'onde de pouls qui, quand il est exécuté par un ordinateur doté d'une unité d'acquisition d'ondes de pouls, lui commande d'exécuter un processus incluant :
l'acquisition d'une onde de pouls d'un corps vivant ;
le calcul d'une pression veineuse sur la base de la forme d'onde de l'onde de pouls sur une période prédéterminée ;
le calcul d'au moins un paramètre vital pour le corps vivant en analysant la forme d'onde de l'onde de pouls dans la période prédéterminée ; et
la sortie de la pression veineuse calculée, du paramètre vital calculé et de l'information sur la précision de la pression veineuse, ladite information étant déterminée sur la base du paramètre vital.
